# EUROPEAN PATENT APPLICATION

(11) **EP 4 183 316 A1**
(43) Date of publication of application: **24.05.2023**
(21) Application number: 22204113.9
(22) Date of filing: 27.10.2022
(51) Int. Cl.: A61B 1/018, A61B 1/00, A61B 17/00, A61B 17/34

(54) **ENDOSCOPE INTERNAL DYNAMIC SEAL**

(30) Priority: 18.11.2021 US 202163280700 P
(71) Applicant: Cook Medical Technologies, LLC, Bloomington, IN 47404 (US)
(72) Inventor: BRECHT, Michael J., Pfafftown, 27040 (US); SPENCER, Marc, Winston-Salem, 27104 (US); CARRUTHERS, Christopher A., Winston-Salem, 27104 (US)
(74) Representative: Leach, Sean Adam

(57) **Abstract**

Disclosed herein is a medical device including a dynamic seal and a movable member. The device allows the movable member to translate proximally and distally through the dynamic seal while fluid does not flow proximally to the dynamic seal member.

## Description

### FIELD

The present disclosure relates to medical devices. More particularly, the disclosure relates to internal seals within endoscope systems.

### BACKGROUND

The statements in this section merely provide background information related to the present disclosure and may not constitute prior art.

Internal body cavities and body lumens may become blocked, or the walls surrounding them may develop growths. In some cases, removal of these blockages or growths, or other treatment thereof, may be necessary. Endoscopic or other minimally invasive techniques may be used to treat these situations.

One type of treatment includes the use of catheters or other endoscopic devices that are inserted into the body lumen or cavity and toward the area where treatment is desired. Insertion of the endoscope to the target area can allow for visualization of the target area and a determination of the desired procedure and the specific location of the area to be treated.

In general, endoscopes have been designed to be operated with the same fundamental mechanisms, and have not had transformational improvements. Endoscopes generally include a camera and a set of wheels that an operator, such as a physician, operates with a first hand (in some cases, the left hand) to control scope deflection, while the second (generally, right) hand switches between the insertion tube of the endoscope and the accessory channel in order to control scope and device advancement, respectively, through the anatomy of a patient.

Specialized endoscopes, tailored to specific procedures, are becoming more common in the field of endoscopy with the trend from reusable scopes to disposable scopes. Whereas traditional endoscopes include accessory channel(s) that are fixed from axial movement, specialized endoscopes may include a feature in which the accessory channels may move proximally or distally so as to change the camera between forward-viewing and side-viewing configurations. Escape of gas from the proximal end of the insertion tube should be minimized, and the escape of gas has traditionally been minimized by use of static seals. Given the proximal or distal movement of accessory channels in some specialized endoscopes, static sealing methods used in traditional endoscopes are not possible between the accessory channels and the insertion tube. Further, in such specialized endoscopes, deflection wires, coils, or cables used to provide deflection of the distal end of the endoscope must be exposed distally to allow the accessory channels to deflect between the bending section linkages. Accordingly, static sealing around the deflection wires is insufficient. Additionally, in such specialized endoscopes, electrical or fiber optic wires are used to provide power, one-way signaling, two-way signaling, communication for data, or light at the distal end of the endoscope to power LED(s), other sensors, circuits, or light, and static sealing around electrical or fiber optic wires is insufficient.

### SUMMARY

In an example, the present disclosure provides a medical device. The medical device includes an elongate tube including a lumen extending therethrough, the elongate tube defining a longitudinal axis therethrough. The medical device further includes a movable member extending longitudinally at least partially within the lumen. The medical device further includes a dynamic seal, including a dynamic seal member at a proximal end of the elongate tube, the movable member configured to translate proximally and distally through the dynamic seal member, and a flexible seal through which the movable member extends longitudinally. The dynamic seal member is configured to prevent fluid from flowing proximally to the dynamic seal member. The flexible seal member may be an elastomeric seal. The flexible seal may include an amorphous sealing material. The movable member may be an accessory channel of an endoscope system. The movable member may be a deflection wire of an endoscope system. The movable member may be an electrical wire or a fiber-optic wire of an endoscope system. The medical device may include a plurality of movable members and the dynamic seal may include a plurality of flexible seals, one movable member of the plurality of movable members each extending extend through one flexible seal of the plurality of flexible seals. The medical device may include a second dynamic seal member wherein the dynamic seal member may be within the second dynamic seal member and the second dynamic seal member may be rotationally decoupled from the dynamic seal member. The dynamic seal member may be variably adjustable and configured to increase or decrease friction on the movable member.

In another example, the present disclosure provides a medical device. The medical device includes an elongate tube including a lumen extending therethrough, the elongate tube defining a longitudinal axis therethrough. The medical device further includes a movable member extending longitudinally at least partially within the lumen. The medical device further includes a first dynamic seal member at a proximal end of the elongate tube, the movable member configured to translate proximally and distally through the first dynamic seal member. The medical device further includes a flexible seal through which the movable member extends longitudinally. The medical device further includes a second dynamic seal member rotationally decoupled from the first dynamic seal member. The first dynamic seal member is within the second dynamic seal member. The first dynamic seal member is configured to prevent fluid from flowing proximally to the first dynamic seal member. The flexible seal may be an elastomeric seal. The flexible seal may include an amorphous sealing material. The movable member may be an accessory channel of an endoscope system. The movable member may be a deflection wire of an endoscope system. The movable member may be an electrical wire or a fiber-optic wire of an endoscope system. The medical device may include a plurality of movable members and the first dynamic seal member may include a plurality of flexible seals, one movable member of the plurality of movable members each extending through one flexible seal of the plurality of flexible seals. The medical device may include a catch proximal to the second dynamic seal member, the second dynamic seal member configured to move between a locked configuration and an unlocked configuration, the second dynamic seal member being biased to the locked configuration. When the second dynamic seal member is in the unlocked configuration, the second dynamic seal member may be rotatable about the longitudinal axis relative to the first dynamic seal member. When the second dynamic seal member is in the locked configuration, the second dynamic seal member may engage with the catch and not be rotatable about the longitudinal axis relative to the first dynamic seal member.

In yet another example, the present disclosure provides a medical device. The medical device includes a lumen extending therethrough, the elongate tube defining a longitudinal axis therethrough. The medical device further includes a movable member extending longitudinally at least partially within the lumen. The medical device further includes a dynamic seal includes a dynamic seal member at a distal end of the elongate tube, the movable member configured to translate proximally and distally through the dynamic seal member and a flexible seal through which the movable member extends longitudinally. The dynamic seal member may be configured to prevent fluid from flowing proximally to the dynamic seal member.

Further areas of applicability will become apparent from the description provided herein. It should be understood that the description and specific examples are intended for purposes of illustration only and are not intended to limit the scope of the present disclosure.

### DRAWINGS

In order that the present disclosure may be well understood, there will not be described various forms thereof, given by way of example, reference being made to the accompanying drawings. The components in the figures are not necessarily to scale. Moreover, in the figures, like-referenced numerals designate corresponding parts through the different views.
FIG. **1** illustrates a perspective view of an example of an endoscope system having a handle constructed according to the principles of the present disclosure;
FIG. **2** illustrates a perspective view of an example of the elements of a dynamic seal, together with movable member(s) and elongate tube, in accordance with the principles of the present disclosure;
FIG. **3** illustrates a perspective view of an example of a dynamic seal as assembled, in accordance with the principles of the present disclosure;
FIG. **4** illustrates a diametrical longitudinal cross-sectional view of an example of a dynamic seal as assembled, in accordance with the principles of the present disclosure;
FIG. **5** illustrates a chordal longitudinal cross-sectional view of an example of a dynamic seal as assembled, in accordance with the principles of the present disclosure;
FIG. **6** illustrates a top view of an example of a middle dynamic seal member in accordance with the principles of the present disclosure;
FIG. **7** illustrates a perspective view of an example of proximal and distal middle dynamic seal members and seals in accordance with the principles of the present disclosure;
**FIG. 8** illustrates the example of the elements of the dynamic seal of FIG. **2** including deflection wires, in accordance with the principles of the present disclosure;
**FIG. 9** illustrates a perspective view of an example of a handle including an example of a dynamic seal with a lower dynamic seal member shown as see-through to show a spring internal to the lower member, in accordance with the principles of the present disclosure; and
FIG. **10** illustrates a perspective view of the example of the handle of FIG. **9** with the lower dynamic seal member shown as see-through, the lower dynamic seal member moved distally away from the upper dynamic seal member to compress the spring internal to the lower dynamic seal member, in accordance with the principles of the present disclosure.

The drawings described herein are for illustration purposes only and are not intended to limit the scope of the present disclosure in any way.

### DETAILED DESCRIPTION

The following description is merely exemplary in nature and is not intended to limit the present disclosure, application, or uses. It should be understood that throughout the drawings, corresponding reference numerals indicate like or corresponding parts and features.

In adding reference denotations to elements of each drawing, although the same elements are displayed on a different drawing, it should be noted that the same elements have the same denotations. In addition, in describing one aspect of the present disclosure, if it is determined that a detailed description of related well-known configurations or functions blurs the gist of one aspect of the present disclosure, it will be omitted.

In the following discussion, the terms "proximal" and "distal" will be used to describe the opposing axial ends of the device, as well as the axial ends of various component features. The term "proximal" is used in its conventional sense to refer to the end of the device (or component) that is closest to the medical professional during use of the assembly. The term "distal" is used in its conventional sense to refer to the end of the device (or component) that is initially inserted into the patient, or that is closest to the patient during use. The term "longitudinal" will be used to refer to an axis that aligns with the proximal-distal axis of the device (or component). The terms "radially" and "radial" will be used to refer to elements, surfaces, or assemblies relative to one another that may extend perpendicularly from a longitudinal axis. The terms "circumference," "circumferentially," and "circumferential" will be used to refer to elements, surfaces, or assemblies relative to one another encircling a longitudinal axis at a radius. The terms "diameter" and "diametrical" refer to a line passing through a circle that touches two points on the circumference of the circle and the center of the circle. The terms "chord" and "chordal" refer to a line passing through a circle that touches two points on the circumference of the circle but does not touch the center of the circle.

The uses of the terms "a" and "an" and "the" and similar referents in the context of describing the present disclosure (especially in the context of the following claims) are to be construed to cover both the singular and the plural, unless otherwise indicated herein or clearly contradicted by context. The use of the term "plurality of" is defined by the Applicant in the broadest sense, superseding any other implied definitions or limitations hereinbefore or hereinafter unless expressly asserted by the Applicant to the contrary, to mean a quantity of more than one. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein the terms "comprise(s)," "include(s)," "having," "has," "can," "contain(s)," and variants thereof, are intended to be open-ended transitional phrases, terms, or words that do not preclude the possibility of additional acts or structures. The present description also contemplates other examples "comprising," "consisting of," "and consisting essentially of," the examples or elements presented herein, whether explicitly set forth or not.

In describing elements of the present disclosure, the terms 1^{st}, 2^{nd}, first, second, A, B, (a), (b), and the like may be used herein. These terms are only used to distinguish one element from another element, but do not limit the corresponding elements irrespective of the nature or order of the corresponding elements.

Unless otherwise defined, all terms herein, including technical or scientific terms, have the same meanings as those generally understood by those skilled in the art to which the present disclosure pertains. Such terms as those defined in a generally used dictionary are to be interpreted as having meanings equal to the contextual meanings in the relevant field of art.

As used herein, the term "about," when used in the context of a numerical value or range set forth means a variation of ±15%, or less, of the numerical value. For example, a value differing by ±15%, ±14%, ±10%, or ±5%, among others, would satisfy the definition of "about," unless more narrowly defined in particular instances.

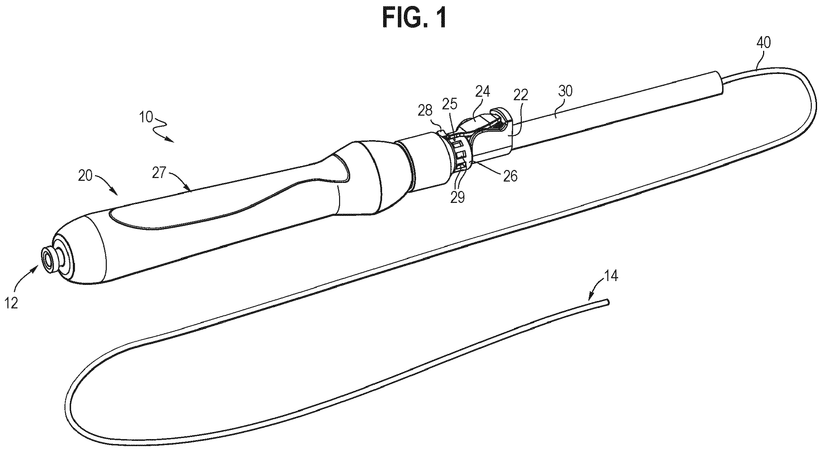

Referring to FIG. **1****,** an example of an endoscope system **10** is illustrated. The endoscope system **10** extends from proximal end **12** to distal end **14,** and includes a handle **20.** The endoscope system **10** also includes an elongate tube **30** which is engaged with the handle **20** such that it can rotate relative to the handle **20.** The elongate tube **30** may be a flexible tube with at least one lumen **38** running throughout its length. In one aspect, the elongate tube **30** may be made of a braided material, such as a polyether block amide (including, for example, PEBAX) with a polytetrafluoroethylene ("PTFE") liner to provide sufficient torqueability and pushability. Other potential materials for the elongate tube **30** include, but are not limited to, polyethylene, polypropylene, and nylon.

The endoscope system may further include a movable member **40** running through the elongate tube **30** and/or the handle **20.** Movable member **40** may be designed as individual elongated tubes that may be movable within the lumen **38** of the elongate tube **30,** thus allowing longitudinal movement of the movable member **40** with respect to the elongate tube **30.** Examples of movable member **40** may include accessory channel(s), deflection wire(s), electrical wire(s), and fiber-optic wire(s). Electrical wire(s) or fiber-optic wires may provide power, one-way signaling, two-way signaling, communication for data, or light at the distal end of the endoscope to power light emitting diode(s) (LED(s)), other sensors, circuits, or light. When the elongate tube **30** is in a straight configuration, elongate tube **30** defines a longitudinal axis running centrally therethrough, which represents the axis about which elongate tube **30** may rotate relative to handle **20.** In practice, even when elongate tube **30** is not in a straight configuration, the longitudinal axis is still defined at the same position, and the portion of elongate tube **30** that is outside of the body of the patient and that substantially surrounds the longitudinal axis will be rotatable about said axis.

While FIG. **1** illustrates single movable member **40,** an endoscope system **10** may include two movable members **40,** three movable members **40,** or more. For example, a single, larger movable member **40** may be used to accommodate larger endoscopic tools. Further, in lieu of individual movable member(s) **40,** a single elongate tube **30** may be used with two or more channel lumens running through it. The movable member(s) **40** may individually range in diameter anywhere from 0.5 millimeter to 20 millimeters, or from 1 millimeter to 10 millimeters. The movable member(s) **40** may extend from proximal of or past handle **20,** through lumen **38,** and through distal end **14.** Movable member(s) **40** may have an open end on both ends, and various tools, devices, and cameras may be inserted into and removed from movable member(s) **40.** While movable member(s) **40** have been illustrated to extend well beyond the end of elongate tube **30** in FIG. **1****,** this drawing is not necessarily to scale, and elongate tube **30** may have a greater length.

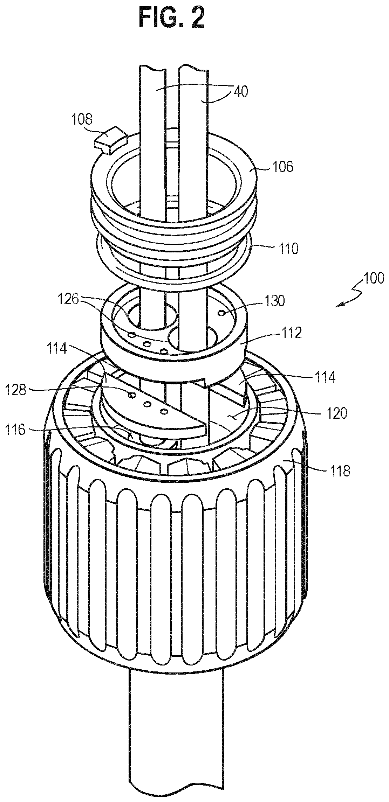

FIG. **2** illustrates a perspective view of an example of the elements of a dynamic seal **100,** together with movable member(s) **40** in the form of two accessory channels, and elongate tube **30.** Movable member(s) **40** such as accessory channel(s) may move proximally or distally to change endoscope system **10** between forward-viewing and side viewing configurations, which requires a dynamic sealing between movable member(s) **40** and elongate tube **30.** Additionally, dynamic sealing is required around deflection wires **402, 404** used to provide deflection of distal end **14** of endoscope system **10.**

As illustrated by FIG. **2****,** sealing between the inner lumen of elongate tube **30** and movable member(s) **40** such as accessory channel(s) or deflection wires **402, 404** may be affected by attachment of lower dynamic seal member **118** around the proximal end of elongate tube **30.** Alternatively, though not shown in the example illustrated in FIG. **2****,** instead of at the proximal end of elongate tube **30,** one or more dynamic seal members may be positioned at the distal end of elongate tube **30** and configured to prevent pressurized fluid from flowing proximally to the dynamic seal while allowing movable member(s) to translate through the dynamic seal in distal and proximal directions. Moveable member(s) **40** are then passed through seal **110,** which is constrained by upper dynamic seal member **106,** upper dynamic seal member **106** proximal to lower dynamic seal member **118.** Movable member(s) **40** also pass through upper dynamic seal member **106.** Upper dynamic seal member **106** may include stop **108** to preclude complete 360-degree rotation about the longitudinal axis by stop **108** confronting, for example, a catch. Middle dynamic seal member **112** may be a seat for seals **110** and **116** and arcuate seals **114** but may be free floating between upper dynamic seal member **106** and lower dynamic seal member **118** such that middle dynamic seal member **112** may freely rotate. Upper dynamic seal member **106** and lower dynamic seal member **118** may rotate about the longitudinal axis, relative to and around middle dynamic seal member **112,** such that middle dynamic seal member **118,** seals **110** and **116** and arcuate seals **114,** movable member(s) **40,** and deflection wires are decoupled from the rotation and remain fixed in position while maintaining sealing. During rotation of upper dynamic seal member **106** and lower dynamic seal member **118,** inner surface **120** of lower dynamic seal member **118** may confront middle dynamic seal member **112.** FIG. **2** illustrates a plurality of seals, including seals **110** and **116** and arcuate seals **114.** Though not shown, in an example of a dynamic seal, a seal may be in the form of a single layer with a specific opening geometry for each of upper dynamic seal member **106,** middle dynamic seal member **112,** and lower dynamic seal member **118.** Alternatively, though not shown, in an example of a dynamic seal, a seal may be in the form such that moveable member(s) **40** of varying diameter and/or geometrical shape may pass through the seal. As illustrated in FIG. **2****,** dynamic seal **100** is positioned at the proximal end of elongate tube 30, but the dynamic seal of the present invention may be placed anywhere along the length of elongate tube **30.** The dynamic seals of the present invention are configured to prevent pressurized fluid from flowing proximally to the dynamic seal while allowing movable member(s) to translate through the dynamic seal in distal and proximal directions.

Middle dynamic seal member **112** includes bore(s) **126** extending longitudinally through middle dynamic seal member **112,** through which movable member(s) **40** may translate in distal and proximal directions. Middle dynamic seal member **112** may be variably adjustable with respect to the dimensions and shape of bore(s) **126** so as to advantageously increase or decrease friction on movable member(s) **40** translating through one or more bores **126** as is necessary, desirable, or preferable. Middle dynamic seal member **112** may include deflection bore(s) **130** extending longitudinally through middle dynamic seal member **112** through which movable member(s) such as deflection wires **402, 404** may translate in distal and proximal directions. Deflection bore(s) **130** are configured to align longitudinally with seal bore(s) **128** in arcuate seals **114,** through which deflection wires **402, 404** may translate in distal and proximal directions. Alternatively, or additionally, though not shown in the example illustrated in FIG. **2****,** movable member(s) may be electrical or fiber optic wires that may pass through deflection bore(s) **130** to provide power, two-way signaling, or light at distal end **14** to power LED(s), other sensors, circuits, or light.

Bore(s) **126** may generally have a larger diameter than deflection bore(s) **130** or seal bore(s) **128.** Alternatively, bore(s) **126** may generally have the same diameter as, or even a smaller diameter than, deflection bore(s) **130** or seal bore(s) **128.** Deflection bore(s) **130** and seal bore(s) **128** may have approximately the same diameter and general size.

Each of seals **110** and **116** and arcuate seals **114** may be formed of flexible silicone, rubber, plastic, elastomer, amorphous sealing material, or any other material suitable to substantially prevent (or at least impede) fluid from flowing across and/or permeating through it. Each of upper dynamic seal member **106,** middle dynamic seal member **112,** or lower dynamic seal member **118** may be formed of nitrile (Buna), neoprene, ethylene propylene diene monomer (EPDM) rubber, silicone, fluorocarbon (Viton), polytetrafluoroethylene (PTFE, Teflon), perfluoroelastomers (FFKM), or any elastomer. Additionally, each of upper dynamic seal member **106,** middle dynamic seal member **112,** or lower dynamic seal member **118** may be encapsulated by an additional material such as fluorinated ethylene propylene (FEP) copolymer.

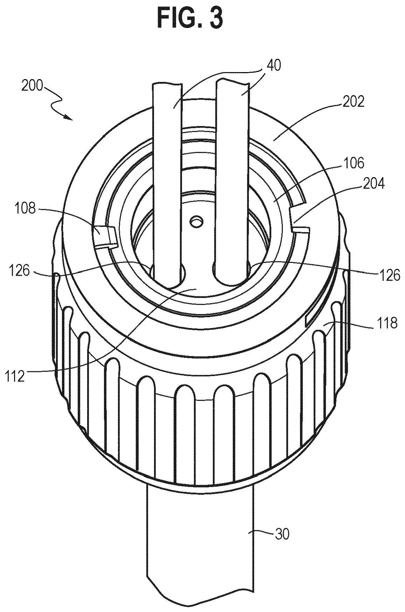

FIG. **3** illustrates a perspective view of an example of a dynamic seal **200** as assembled. Proximal to lower dynamic seal member **118** is catch **202.** Stop **108** of upper dynamic seal member **106** may confront protrusion **204** on catch **202,** to preclude complete 360-degree rotation of upper dynamic seal member **106** and lower dynamic seal member **118** about the longitudinal axis relative to middle dynamic seal members, accessory channels, seals, and deflection wires. Dynamic seal **200** includes a seal or a plurality of seals configured to prevent fluid, including pressurized fluid, from flowing proximally to upper dynamic seal member **106** while movable member(s) **40** may translate in proximal or distal directions. In examples of the present disclosure, movable member(s) **40** may include a single movable member or a plurality of movable members, as is illustrated in FIG. **3****,** which illustrates two movable members **40.** In certain examples, dynamic seal may include a seal deployed to prevent fluid from flowing proximally to upper dynamic seal member **106** while a single movable member, or, alternatively, a plurality of movable members, may translate in proximal or distal directions. In other examples, a dynamic seal may include a plurality of seals deployed to prevent fluid from flowing proximally to upper dynamic seal member **106** while a single movable member, or, alternatively, a plurality of movable members, may translate in proximal or distal directions.

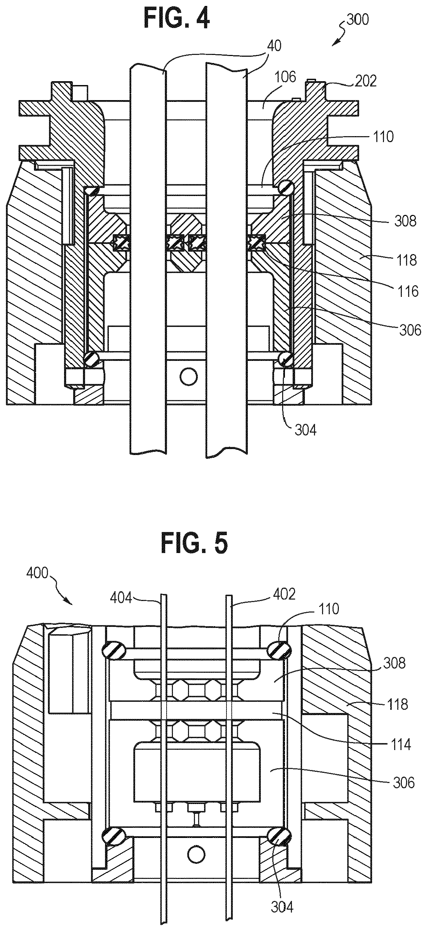

FIG. **4** illustrates a diametrical longitudinal cross-sectional view of an example of a dynamic seal **300** as assembled, with the diametrical longitudinal cross-section through the diameter of proximal middle dynamic seal member **308** and distal middle dynamic seal member **306** that includes the diameters of bores **126** and the diameters of movable members **40.** As illustrated by FIG. **4****,** dynamic seal **300** includes upper dynamic seal member **106,** which is positioned inside catch **202** and through which movable members **40** extend longitudinally. Seal **110** is seated longitudinally between upper dynamic seal member **106** and proximal middle dynamic seal member **308.** Encircling each movable member **40** is a seal **116,** which is seated within complementing recesses in the distal surface of proximal middle dynamic seal member **308** and complementing recesses in the proximal surface of distal middle dynamic seal member **306.** Seal **304** is seated longitudinally between distal middle dynamic seal member **306** and the proximal end of elongate tube **30.** Lower dynamic seal member **118** encircles proximal middle dynamic seal member **308,** distal middle dynamic seal member **306,** seals **110, 116, 304,** and movable members **40,** and lower dynamic seal member **118** and upper dynamic seal member **106** may be rotationally decoupled such that proximal middle dynamic seal member **308,** distal middle dynamic seal member **306,** seals **110, 116, 304,** and movable members **40** remain stationary when lower dynamic seal member **118** and upper dynamic seal member **106** are rotated. Seals **110, 116,** and **304** are positioned, and thereby configured, to prevent fluid, including pressurized fluid, from flowing proximally to upper dynamic seal member **106,** while movable members **40** may translate proximally and distally.

FIG. **5** illustrates a chordal longitudinal cross-sectional view of an example of a dynamic seal **400** as assembled, with the chordal longitudinal cross-section parallel to the cross-section taken to illustrate FIG. **4****,** and through arcuate seal **114.** Deflection wires **402, 404** are movable members passing through proximal middle dynamic seal member **308,** arcuate seal **114,** and distal middle dynamic seal member **306,** with arcuate seal **114** seated in complementary recesses in distal surface of proximal middle dynamic seal member **308** and proximal surface of distal middle dynamic seal member **306.** Arcuate seal **114** is positioned, and thereby configured, to prevent fluid, including pressurized fluid, from flowing proximally to upper dynamic seal member **106,** while deflection wires **402, 404** may translate proximally and distally.

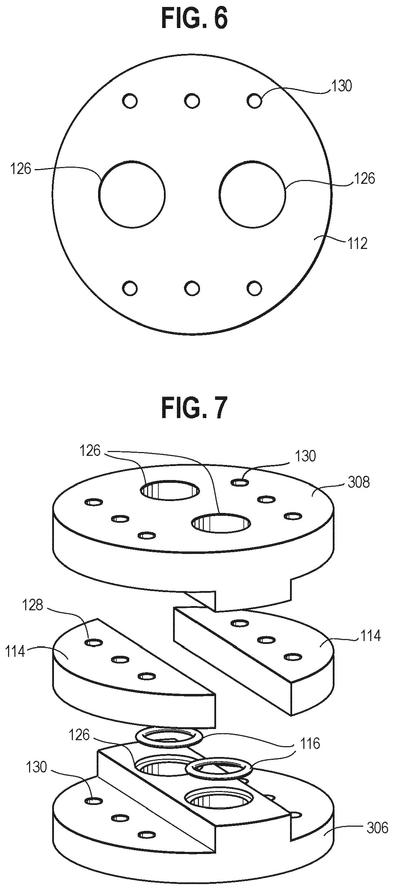

FIG. **6** illustrates a top view of an example of middle dynamic seal member **112.** Middle dynamic seal member **112** includes a plurality of bores **126** extending longitudinally through middle dynamic seal member **112,** and each of which through movable members **40** that are accessory channels may translate proximally or distally. Middle dynamic seal member **112** includes a plurality of deflection bores **130** extending longitudinally through middle dynamic seal member **112,** and each of which through a movable member that is a deflection wire, such as deflection wire **402** or deflection wire **404,** may translate proximally or distally.

FIG. **7** illustrates a perspective view of an example of proximal middle dynamic seal member **308,** distal middle dynamic seal member **306,** arcuate seals **114,** and seals **116** as they may be disposed for assembly in an example of a dynamic seal according to the principles of the present disclosure. Proximal middle dynamic seal member **308** and distal middle dynamic seal member **306** include a plurality of bores **126** extending longitudinally and coaxially through proximal middle dynamic seal member **308** distal middle dynamic seal member **306** such that when the elements illustrated in FIG. **7** are assembled in a dynamic seal, each of the plurality of bores **126** in proximal middle dynamic seal member **308** lines up with a corresponding bore **126** in distal middle dynamic seal member **306** such that a movable member **40** such as an accessory channel may translate proximally and distally through a bore **126** in proximal middle dynamic seal member **308** and a corresponding bore **126** in distal middle dynamic seal member **306.** Proximal middle dynamic seal member **308** and distal middle dynamic seal member **306** also each includes a plurality of deflection bores extending longitudinally through proximal middle dynamic seal member **308** and distal middle dynamic seal member **306** such that when the elements illustrated in FIG. **7** are assembled in a dynamic seal, each of the plurality of deflection bores **130** in proximal middle dynamic seal member **308** lines up with a corresponding deflection bore **130** in distal middle dynamic seal member **306** such that a movable member such as a deflection wire **402** or **404** may translate proximally and distally through a deflection bore **130** in proximal middle dynamic seal member **308** and a corresponding deflection bore **130** in distal middle dynamic seal member **306.** Each arcuate seal **114** includes a plurality of seal bores **128,** and arcuate seals **114** may be seated and compressed between recesses in the distal surface of proximal middle dynamic seal member **308** and the proximal surface of distal middle dynamic seal member **306** such that when the elements illustrated in FIG. 7 are assembled in a dynamic seal, each of the plurality of deflection bores **130** in proximal middle dynamic seal member **308** lines up with a corresponding seal bore **128** in an arcuate seal **114** and with a corresponding deflection bore **130** in distal middle dynamic seal member **306** such that a movable member such as a deflection wire **402** or **404** may translate proximally and distally through each of a plurality of deflection bores **130** in proximal middle dynamic seal member **308,** a corresponding seal bore **128** in arcuate seal **114,** and a corresponding deflection bore **130** in distal middle dynamic seal member **306.** Seals **116** are flexible and seated in each bore **126** in slight recesses in the distal surface of proximal middle dynamic seal member **308** and the proximal surface of distal middle dynamic seal member **306** such that seals **116** are compressed between proximal middle dynamic seal member **308** and distal middle dynamic seal member **306** when the elements illustrated in FIG. **7** are assembled in a dynamic seal.

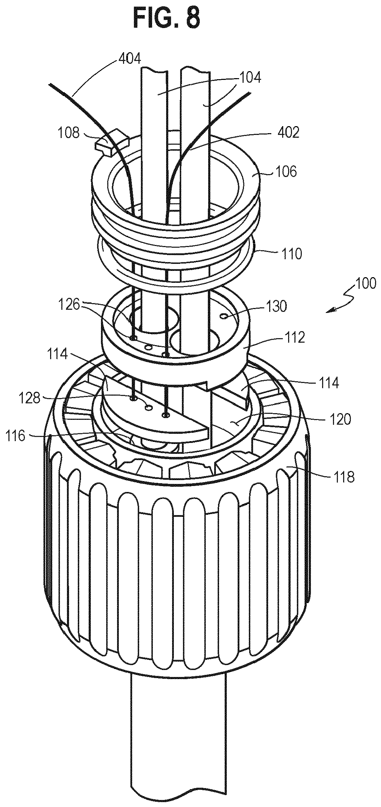

FIG. **8** illustrates the example of the elements of the dynamic seal **100** of FIG. **2** including deflection wires **402** and **404,** each of which pass through upper dynamic seal member **106,** seal **110,** deflection bores **130** in middle dynamic seal member **112,** and seal bores **130** in arcuate seals **114.** Dynamic seal **100** is configured such that deflection wires **402** and **404** may translate distally and proximally while fluid, including pressurized fluid, does not flow proximally to upper dynamic seal member **106.**

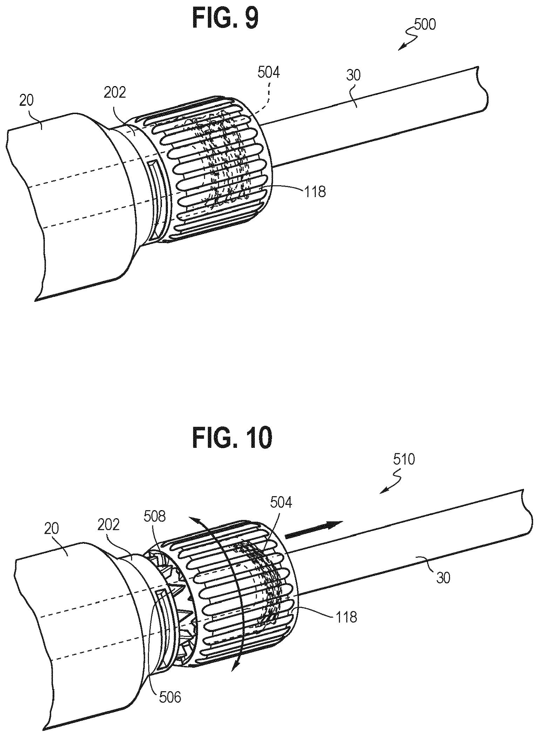

**FIG. 9** illustrates a perspective view of an example of a handle **20** including an example of a dynamic seal **500** with a lower dynamic seal member **118** shown as see-through or transparent for purposes of illustration to show a spring **504** within lower dynamic seal member **118.** Lower dynamic seal member **118** may translate distally relative to catch **202** and handle **20,** but is biased to the locked configuration in which lower dynamic seal member **118** does not translate distally. In the locked configuration, elongate tube **30** is not rotatable about the longitudinal axis relative to handle **20.** Spring **504** is positioned about the circumference internal to lower dynamic seal member **118,** and biases lower dynamic seal member **118** into the locked configuration with proximal end of lower dynamic seal member **118** fronting distal end of catch **202.**

FIG. **10** illustrates a perspective view of the example of the handle **20** of FIG. **9** with the lower dynamic seal member **118** shown as see-through or transparent, the lower dynamic seal member **118** moved distally away from catch **202** to compress spring **504** internal to lower dynamic seal member **118.** When lower dynamic seal member **118** is translated distally, lower dynamic seal member **118** is in an unlocked configuration, in which a plurality of dynamic seal member teeth **508** that may be disposed about the proximal inner surface of lower dynamic seal member **118** are disengaged from a plurality of catch teeth **506,** and spring **504** is compressed. When lower dynamic seal member **118** is in an unlocked configuration, an operator may rotate lower dynamic seal member **118** circumferentially by applying torque, thereby rotating elongate tube **30** relative to catch **202.** Plurality of catch teeth **506** may be disposed about distal circumference of catch **202.** When the operator releases lower dynamic seal member **118,** spring **504** is released, thereby moving lower dynamic seal member **118** proximally back into the locked configuration as illustrated in FIG. **9****.** Plurality of dynamic seal member teeth **508** have a mating geometry with plurality of catch teeth **506** such that in the locked configuration, plurality of dynamic seal member teeth **508** mesh with plurality of catch teeth **506** so as to prevent rotation of elongate tube **30** about the longitudinal axis relative to handle **20.**

A handle with a locking mechanism as described herein may be used in a number of medical device systems, particularly endoscopes. In particle, the handle of the present disclosure may be used in conjunction with the scopes, devices, and systems described in United States Patent Application Serial No. 15/445,318 and United States Patent Application Serial No. 15/445,518, the entire contents of both of which are hereby incorporated by reference herein in their entireties.

Although the present disclosure has been described with reference to examples and the accompanying drawings, the present disclosure is not limited thereto, but may be variously modified and altered by those skilled in the art to which the present disclosure pertains without departing from the spirit and scope of the present disclosure.

The subject-matter of the disclosure may also relate, among others, to the following aspects:

A first aspect relates to a medical device, comprising: an elongate tube comprising a lumen extending therethrough, the elongate tube defining a longitudinal axis therethrough; a movable member extending longitudinally at least partially within the lumen; and a dynamic seal, comprising: a dynamic seal member at a proximal end of the elongate tube, the movable member configured to translate proximally and distally through the dynamic seal member; and a flexible seal through which the movable member extends longitudinally; and wherein the dynamic seal member is configured to prevent fluid from flowing proximally to the dynamic seal member.

A second aspect relates to the medical device of aspect **1,** wherein the flexible seal is an elastomeric seal.

A third aspect relates to the medical device of any preceding aspect, wherein the flexible seal comprises an amorphous sealing material.

A fourth aspect relates to the medical device of any preceding aspect, wherein the movable member is an accessory channel of an endoscope system.

A fifth aspect relates to the medical device of any one of aspects **1** to **3,** wherein the movable member is a deflection wire of an endoscope system.

A sixth aspect relates to the medical device of any one of aspects **1** to **3,** wherein the movable member is an electrical wire or a fiber-optic wire of an endoscope system.

A seventh aspect relates to the medical device of any preceding aspect, comprising a plurality of movable members, wherein the dynamic seal comprises a plurality of flexible seals; and wherein one movable member of the plurality of movable members each extends through one flexible seal of the plurality of flexible seals.

An eighth aspect relates to the medical device of any preceding aspect, further comprising a second dynamic seal member; wherein the dynamic seal member is within the second dynamic seal member; and wherein the second dynamic seal member is rotationally decoupled from the dynamic seal member.

A ninth aspect relates to the medical device of any preceding aspect, wherein the dynamic seal member is variably adjustable and configured to increase or decrease friction on the movable member.

A tenth aspect relates to a medical device, comprising: an elongate tube comprising a lumen extending therethrough, the elongate tube defining a longitudinal axis therethrough; a movable member extending longitudinally at least partially within the lumen; a first dynamic seal member at a proximal end of the elongate tube, the movable member configured to translate proximally and distally through the first dynamic seal member; a flexible seal through which the movable member extends longitudinally; and a second dynamic seal member rotationally decoupled from the first dynamic seal member; wherein the first dynamic seal member is within the second dynamic seal member; and wherein the first dynamic seal member is configured to prevent fluid from flowing proximally to the first dynamic seal member.

An eleventh aspect relates to the medical device of aspect **10,** wherein the flexible seal is an elastomeric seal.

A twelfth aspect relates to the medical device of any one of aspects **10** or **11** wherein the flexible seal comprises an amorphous sealing material.

A thirteenth aspect relates to the medical device of any one of aspects **10** to **12,** wherein the movable member is an accessory channel of an endoscope system.

A fourteenth aspect relates to the medical device of any one of aspects **10** to **12,** wherein the movable member is a deflection wire of an endoscope system.

A fifteenth aspect relates to the medical device of any one of aspects **10** to **12,** wherein the movable member is an electrical wire or a fiber-optic wire of an endoscope system.

A sixteenth aspect relates to the medical device of any one of aspects **10** to **15,** comprising a plurality of movable members; wherein the first dynamic seal member comprises a plurality of flexible seals; and wherein one movable member of the plurality of movable members each extends through one flexible seal of the plurality of flexible seals.

A seventeenth aspect relates to the medical device of any one of aspects **10** to **16,** further comprising a catch proximal to the second dynamic seal member; and wherein the second dynamic seal member is configured to move between a locked configuration and an unloacked configuration, the second dynamic seal member being biased to the locked configuration.

An eighteenth aspect relates to the medical device of aspect **17,** wherein, when the second dynamic seal member is in the unlocked configuration, the second dynamic seal member is rotatable about the longitudinal axis relative to the first dynamic seal member.

A nineteenth aspect relates to the medical device of aspect **17,** wherein, when the second dynamic seal member is in the locked configuration, the second dynamic sela member engages with the catch and is not rotatable about the longitudinal axis relative to the first dynamic seal member.

A twentieth aspect relates to a medical device, comprising: an elongate tube comprising a lumen extending therethrough, the elongate tube defining a longitudinal axis therethrough; a movable member extending longitudinally at least partially within the lumen; and a dynamic seal, comprising: a dynamic seal member at a distal end of the elongate tube, the movable member configured to translate proximally and distally through the dynamic seal member; and a flexible seal through which the movable member extends longitudinally; and wherein the dynamic seal member is configured to prevent fluid from flowing proximally to the dynamic seal member.

In addition to the features mentioned in each of the independent aspects enumerated above, some examples may show, alone or in combination, the optional features mentioned in the dependent aspects and/or as disclosed in the description above and shown in the figures.

## Claims

1. A medical device, comprising:
an elongate tube comprising a lumen extending therethrough, the elongate tube defining a longitudinal axis therethrough;
a movable member extending longitudinally at least partially within the lumen; and
a dynamic seal, comprising:
a dynamic seal member at an end of the elongate tube, the movable member configured to translate proximally and distally through the dynamic seal member; and
a flexible seal through which the movable member extends longitudinally; and
wherein the dynamic seal member is configured to prevent fluid from flowing proximally to the dynamic seal member.

2. The medical device of claim 1, comprising a plurality of movable members;
wherein the dynamic seal comprises a plurality of flexible seals; and
wherein one movable member of the plurality of movable members each extends through one flexible seal of the plurality of flexible seals.

3. The medical device of any preceding claim, further comprising a second dynamic seal member;
wherein the dynamic seal member is within the second dynamic seal member; and
wherein the second dynamic seal member is rotationally decoupled from the dynamic seal member.

4. The medical device of any preceding claim, wherein the dynamic seal member is variably adjustable and configured to increase or decrease friction on the movable member.

5. A medical device, comprising:
an elongate tube comprising a lumen extending therethrough, the elongate tube defining a longitudinal axis therethrough;
a movable member extending longitudinally at least partially within the lumen;
a first dynamic seal member at a proximal end of the elongate tube, the movable member configured to translate proximally and distally through the first dynamic seal member;
a flexible seal through which the movable member extends longitudinally; and
a second dynamic seal member rotationally decoupled from the first dynamic seal member;
wherein the first dynamic seal member is within the second dynamic seal member; and
wherein the first dynamic seal member is configured to prevent fluid from flowing proximally to the first dynamic seal member.

6. The medical device of any preceding claim, wherein the flexible seal is an elastomeric seal.

7. The medical device of any preceding claim, wherein the flexible seal comprises an amorphous sealing material.

8. The medical device of any preceding claim, wherein the movable member is an accessory channel of an endoscope system.

9. The medical device of any preceding claim, wherein the movable member is a deflection wire of an endoscope system.

10. The medical device of any preceding claim, wherein the movable member is an electrical wire or a fiber-optic wire of an endoscope system.

11. The medical device of claim **5,** or any of claims 6 to 10 as dependent thereon, comprising a plurality of movable members;
wherein the first dynamic seal member comprises a plurality of flexible seals; and
wherein one movable member of the plurality of movable members each extends through one flexible seal of the plurality of flexible seals.

12. The medical device of claim **5,** or any of claims 6 to 11 as dependent thereon, further comprising a catch proximal to the second dynamic seal member; and
wherein the second dynamic seal member is configured to move between a locked configuration and an unlocked configuration, the second dynamic seal member being biased to the locked configuration.

13. The medical device of claim **12,** wherein, when the second dynamic seal member is in the unlocked configuration, the second dynamic seal member is rotatable about the longitudinal axis relative to the first dynamic seal member.

14. The medical device of claim **12 or 13,** wherein, when the second dynamic seal member is in the locked configuration, the second dynamic seal member engages with the catch and is not rotatable about the longitudinal axis relative to the first dynamic seal member.

15. The medical device of any of claims 1 to 4 or any of claims 6 to 10 as dependent thereon wherein the dynamic seal member is at one of (a) a proximal end of the elongate tube; and (b) a distal end of the elongate tube.
